(19) Europäisches Patentamt
European Patent Office
Office européen des brevets

(11) Veröffentlichungsnummer: **0 049 682**
**B1**

(12) # EUROPÄISCHE PATENTSCHRIFT

(45) Veröffentlichungstag der Patentschrift: **04.04.84**

(51) Int. Cl.³: **C 07 F 7/22**, A 01 N 55/04, C 07 D 251/58, C 07 D 251/70, C 07 D 251/52

(21) Anmeldenummer: **81810367.3**

(22) Anmeldetag: **07.09.81**

(54) **Triorganozinngruppen enthaltende Triazinverbindungen.**

(30) Priorität: **12.09.80 CH 6882/80**

(43) Veröffentlichungstag der Anmeldung: **14.04.82 Patentblatt 82/15**

(45) Bekanntmachung des Hinweises auf die Patenterteilung: **04.04.84 Patentblatt 84/14**

(84) Benannte Vertragsstaaten: **AT BE CH DE FR GB IT LI NL**

(56) Entgegenhaltungen:
**DE - A - 2 054 483**
**GB - A - 859 143**

(73) Patentinhaber: **CIBA-GEIGY AG, Postfach, CH-4002 Basel (CH)**

(72) Erfinder: **Wehner, Wolfgang, Dr., Wetzbach 34, D-6144 Zwingenberg (DE)**
Erfinder: **Farooq, Saleem, Dr., Im Schalengarten 2, CH-4107 Ettingen (CH)**
Erfinder: **Köstler, Hans-Günter, Gerhart-Hauptmannstrasse 11, D-6148 Heppenheim/Bergstrasse (DE)**

**EP 0 049 682 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Triorganozinngruppen enthaltende Triazinverbindungen

Die vorliegende Erfindung betrifft Triorganozinn-Triazinverbindungen, Verfahren zu ihrer Herstellung und ihre Verwendung in der Schädlingsbekämpfung.

Die Triorganozinn-Triazinverbindungen haben die Formel

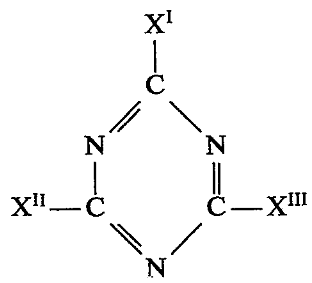

(I)

worin

$X^I$ $-S-Y-COOSn(R')_3$, $-O-Y-COOSn(R')_3$ oder $-NH-Y-COOSn(R')_3$,
$X^{II}$ und $X^{III}$ je $X^I$, Halogen, $(C_1-C_6\text{-Alkyl})_2N-$, $(C_1-C_6\text{-Alkyl})-NH-$, $(C_1-C_6\text{-Alkyl})-O-$ oder $(C_1-C_6\text{-Alkyl})-S-$,
Y $C_1-C_6$-Alkylen und die Reste
R′ gleich oder verschieden sind und $C_1-C_{12}$-Alkyl oder gegebenenfalls substituiertes Benzyl, Phenyl oder Cyclohexyl bedeuten.

Als Substituenten an den Benzyl, Phenyl oder Cyclohexyl bei R′ kommen Halogen, $C_1-C_4$-Alkyl, $C_1-C_4$-Alkoxy oder Trifluormethyl in Frage. Unter Halogen ist Fluor, Chlor, Brom oder Jod, insbesondere aber Chlor, zu verstehen. Die bei $X^{II}$, $X^{III}$ und R′ stehenden Alkylgruppen können geradkettig oder verzweigt sein. Beispiele solcher Gruppen sind u. a.: Methyl, Äthyl, Propyl, Isopropyl, n-, i-, sek.- oder tert.-Butyl, n-Pentyl, n-Hexyl, n-Decyl oder n-Dodecyl sowie deren Isomere. Bevorzugt sind Methyl, Äthyl, Isopropyl oder Propyl.

Die bei Y in Frage kommenden Alkylengruppen können geradkettig oder verzweigt sein. Das bevorzugte Beispiel dieser Gruppe ist Methylen.

Bevorzugt sind Verbindungen der Formel I, worin

$X^I$ $-S-Y-COOSn(R')_3$,
$X^{II}$ und $X^{III}$ je $(C_1-C_6\text{-Alkyl})NH-$,
Y Methylen und
R′ n-Butyl, Phenyl oder Cyclohexyl bedeuten.

Besonders bevorzugt sind Verbindungen der Formel I, worin

$X^I$ $-S-Y-COOSn(R')_3$,
$X^{II}$ und $X^{III}$ je $(C_1-C_6\text{-Alkyl})NH-$,
Y Methylen und
R′ Cyclohexyl bedeuten.

Die Verbindungen der Formel I können nach an sich bekannten Methoden z. B. wie folgt hergestellt werden:

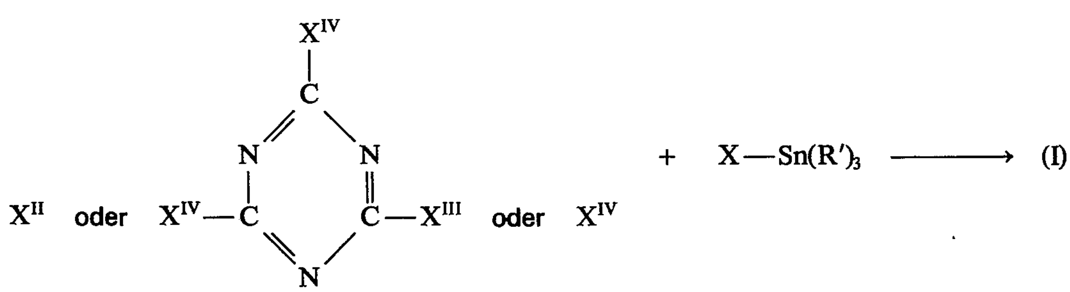

(II) (III)

In den Formeln II und III haben $X^{II}$, $X^{III}$ und R′ die für die Formel I angegebene Bedeutung, $X^{IV}$ steht für

$-S-Y-COOH$, $-O-Y-COOH$ oder $-NH-Y-COOH$, worin Y $C_1-C_6$-Alkylen bedeutet, und X für eine $(R')_3SnO$- oder HO-Gruppe oder ein Halogenatom, insbesondere für Chlor. Falls X ein Halogenatom darstellt, muß die Reaktion in Gegenwart einer Base als Halogenwasserstoffakzeptor durchgeführt werden. Besonders bevorzugt sind solche Verbindungen der Formel II, welche die Reste $X^{II}$, $X^{III}$ und $X^{IV}$ aufweisen.

Als geeignete Basen kommen insbesondere tertiäre Amine, wie Trialkylamine, Pyridine und Dialkylaniline, ferner Hydroxide, Oxide, Carbonate und Bicarbonate von Alkali- und Erdalkalimetallen sowie Alkalimetallalkoholate wie z. B. Kalium-tert.butylat und Natrium-methylat in Betracht.

Die Verfahren werden zweckmäßig bei einer Temperatur zwischen $-10$ bis 150°C, vorzugsweise zwischen 20 bis 80°C, bei normalem oder leicht erhöhtem Druck (bis 5 bar) und vorzugsweise in Gegenwart eines gegenüber den Reaktionsteilnehmern inerten Lösungs- oder Verdünnungsmittels durchgeführt.

Als Lösungs- oder Verdünnungsmittel eignen sich z. B. Äther und ätherartige Verbindungen wie Diäthyläther, Di-isopropyläther, Dioxan und Tetrahydrofuran; aromatische oder aliphatische Kohlenwasserstoffe wie Pentan, Hexan, Dekalin, Benzol, Toluol und Xylole; und Ketone wie Aceton, Methyläthylketon und Cyclohexanon.

Die Ausgangsstoffe der Formel III sind bekannt und können nach bekannten Methoden hergestellt werden. Die Ausgangsstoffe der Formel II sind neu. Sie können nach bekannten Methoden hergestellt werden und bilden ebenfalls einen Gegenstand der vorliegenden Erfindung.

Die Verbindungen der Formel I eignen sich zur Bekämpfung von verschiedenartigen Schädlingen an Tieren und Pflanzen. So können sie zur Bekämpfung aller Entwicklungsstadien von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera eingesetzt werden. Ferner weisen sie auch eine gute fungizide Wirkung auf. Vor allem eignen sich Verbindungen der Formel I jedoch zur Bekämpfung von phytopathogenen und tierparasitären Milben und von Zecken der Ordnung Acarina.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien, z. B. Calcit oder Sand, in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}-C_{22}$), wie z. B. die Na- oder K-Salze der Öl- oder Stearinsäure, oder von natürlichen Fettsäuregemischen, die z. B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls

substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxyd-Addukten. Die sulfonierten Benzimidazolderivate enthalten vorzugsweise 2 Sulfonsäuregruppen und einen Fettsäurerest mit 8—22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkonzentrationsproduktes.

Ferner kommen auch entsprechende Phosphate wie z. B. Salze des Phosphorsäureesters eines p-Nonylphenol-(4—14)-Äthylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben:

»McCutcheon's Detergents and Emulsifiers Annual«, MC Publishing Corp., Ringwood, New Jersey, 1980.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

1. Emulsions-Konzentrate

| | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | – | – |
| Tributylphenyl-polyäthylenglykoläther (30 Mol AeO) | – | 12% | 4,2% |
| Cyclohexanon | – | 15% | 20% |
| Xylolgemisch | 70% | 25% | 20% |

Aus solchen Konzentraten können durch Verdünnen mit Wasser Emulsionen jeder gewünschten Konzentration hergestellt werden.

2. Lösungen

| | a) | b) | c) | d) |
|---|---|---|---|---|
| Wirkstoff | 80% | 10% | 5% | 95% |
| Äthylenglykol-monomethyl-äther | 20% | – | – | – |
| Polyäthylenglykol M G 400 | – | 70% | – | – |
| N-Methyl-2-pyrrolidon | – | 20% | – | – |
| Epoxydiertes Kokosnußöl | – | – | 1% | 5% |
| Benzin (Siedegrenzen 160–190°C) | – | – | 94% | – |

Die Lösungen sind zur Anwendung in Form kleinster Tropfen geeignet.

3. Granulate

| | a) | b) |
|---|---|---|
| Wirkstoff | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschließend im Vakuum abgedampft.

4. Stäubemittel

| | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I

(%= Gewichtsprozent)

5. Spritzpulver

| | a) | b) |
|---|---|---|
| Wirkstoff | 20% | 60% |
| Na-Ligninsulfonat | 5% | 5% |
| Na-Laurylsulfat | 3% | – |
| Na-Diisobutylnaphthalinsulfonat | – | 6% |
| Octylphenolpolyäthylenglykoläther (7–8 Mol AeO) | – | 2% |
| Hochdisperse Kieselsäure | 5% | 27% |
| Kaolin | 67% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschließend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

10. Suspensions-Konzentrat

| | |
|---|---|
| Wirkstoff | 40% |
| Äthylenglkyol | 10% |
| Nonylphenolpolyäthylenglykoläther (15 Mol AeO) | 6% |
| Na-Lingninsulfonat | 10% |
| Carboxymethylcellulose | 1% |
| 37%ige wäßrige Formaldehyd-Lösung | 0,2% |
| Silikonöl in Form einer 75%igen wäßrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

a) Herstellung des Ausgangsproduktes der Formel

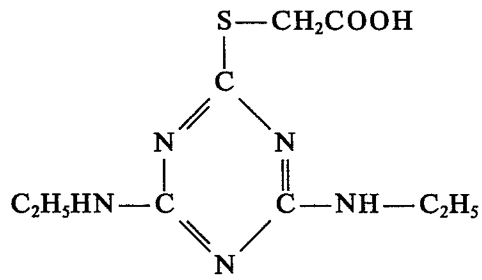

Zu 60,5 g Simazin gelöst in 500 ml Dimethylformamid läßt man bei 70° C 27,7 g Thioglykolsäure und anschließend 60,7 g Triäthylamin tropfen. Nach 2 Stunden Rühren bei 70° C entfernt man die flüchtigen Bestandteile im Vakuum, nimmt den Rückstand in 500 ml Äthyläther auf, filtriert vom Niederschlag ab und erhitzt das Filtrat während einer Stunde auf 80°C ($10^{-2}$ mb). Man erhält nach dem Umkristallisieren aus Methanol/Wasser (5 : 1) die Titelverbindung mit einem Schmelzpunkt von 104—106° C.

b) Herstellung des Endproduktes der Formel

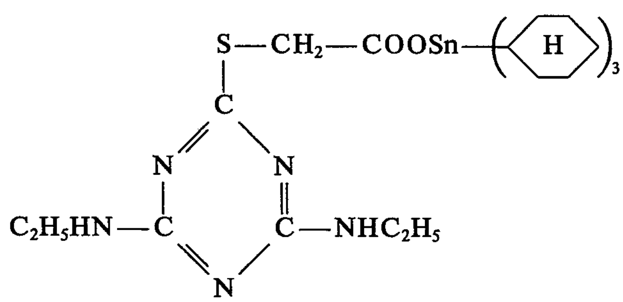

(Verbindung A)

25,7 g der Verbindung der Formel

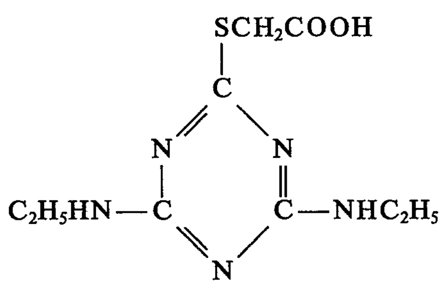

und 35,7 g Tricyclohexylzinnhydroxid werden in 100 ml Toluol zum Rückfluß erhitzt, wobei das Reaktionswasser entfernt wird. Nach dem Entfernen der flüchtigen Bestandteile im Vakuum verbleibt ein viskoses Öl, das beim Abkühlen erstarrt. Man erhält 59,1 g der Verbindung A, die nach dem Umkristallisieren aus Isopropanol einen Schmelzpunkt von 116–118°C aufweist.

Auf analoge Weise werden auch folgende Verbindungen hergestellt:

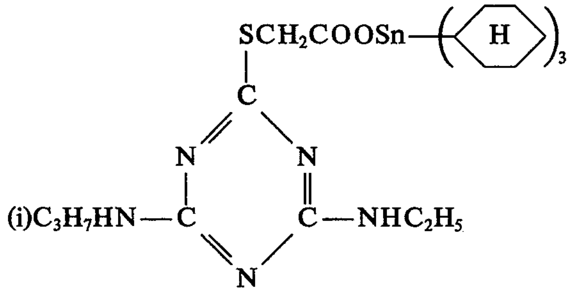

(Verbindung B)

HMR-Spektrum im $CDCl_3$

| Proton | δ (ppm) | Intensität |
|---|---|---|
| $-NH-CH_2-$ | 5,0 (breit) | 2 |
| 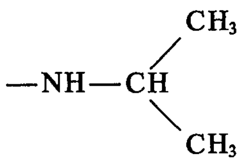 | 4,2 (m) | 1 |
| $-NH-CH_2-CH_3-$ | 3,3 (m) | 2 |
| $-S-CH_2-CO_2$ | 3,1 (s) | 2 |
| $C-C_6H_{11}$ (Signalgruppe) | | |

IR-Spektrum in $CDCl_3$: $\nu_{CD}$: 1550 $cm^{-1}$ (Salzbande).

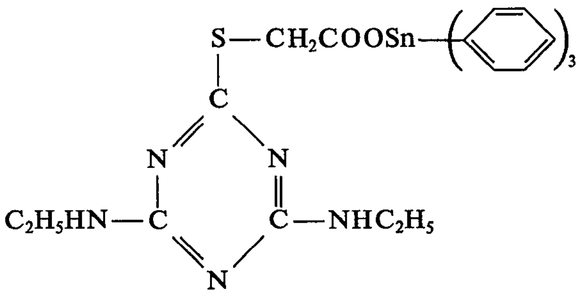

(Verbindung C)

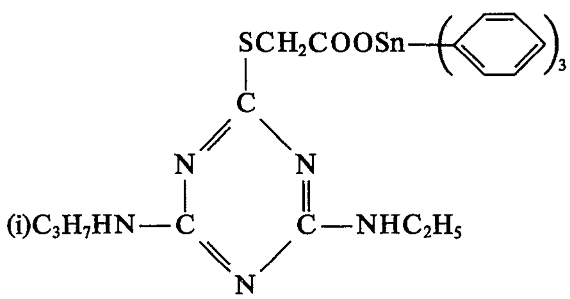

(Verbindung D)

Smp. 77–81°C

Beispiel 2

a) Herstellung des Ausgangsproduktes der Formel

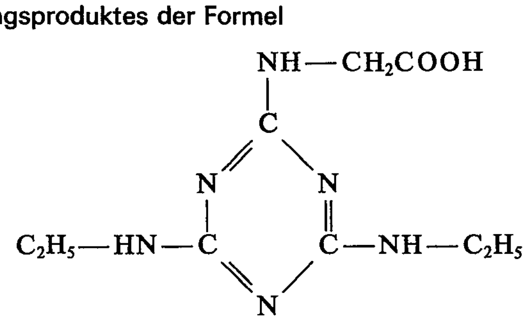

45 g Glycin und 121 g Triäthylamin werden in 800 ml Dimethylacetat bei 70°C vorgelegt. Unter Rühren trägt man portionenweise 101 g Simazin ein. Anschließend wird 24 Stunden bei 140°C erhitzt. Nach dem Abkühlen werden die Feststoffe abfiltriert. Das klare Filtrat wird zuerst bei 20°C mit 250 ml konz. Salzsäure und anschließend bis zur deutlichen alkalischen Reaktion mit Natronlauge versetzt. Nach der Filtration vom schmierig braunen Niederschlag wird mit Eisessig bis zur schwach sauren Reaktion versetzt. Der farblose Niederschlag wird abfiltriert und getrocknet. Man erhält die Titelverbindung als kristallines Pulver, dessen HMR-Spektrum mit der Struktur in Einklang steht.

b) Herstellung des Ausgangsproduktes der Formel

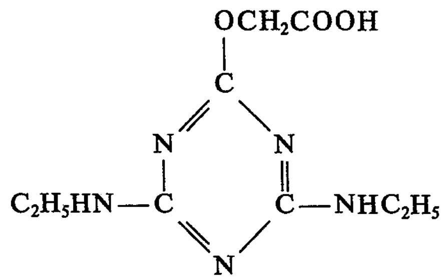

45 g Glycin und 121 g Triäthylamin werden in 800 ml Dimethylacetat bei 70° C vorgelegt. Unter Rühren trägt man portionenweise 101 g Simazin ein. Anschließend wird 24 Stunden bei 140° C erhitzt. Nach dem Abkühlen werden die Feststoffe abfiltriert. Das bei 20° C mit 400 ml konz. Salzsäure und mit 1 l Wasser versetzte Filtrat wird mit Natronlauge alkalisch gestellt und filtriert. Nach dem Ansäuern mit Eisessig und dem Einengen wird das Filtrat in 150 ml Chloroform gelöst, 3mal mit Wasser ausgeschüttelt, getrocknet und mit Chloroform als Eluiermittel an Kieselgel chromatographiert. Man erhält die Titelverbindung als hellgelbes Öl, dessen HMR-Spektrum mit der Struktur in Einklang steht.

c) Herstellung der Endprodukte

0,3 g der Verbindung der Formel

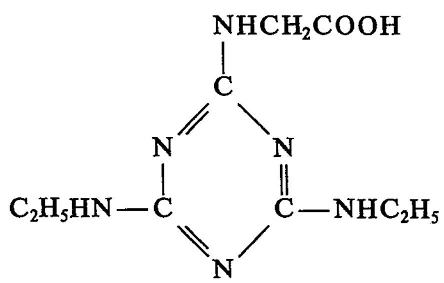

und 4,8 g Tricyclohexylzinnhydroxid werden in 50 ml Toluol zum Rückfluß erhitzt, wobei das Reaktionswasser abgetrennt wird. Die Lösung wird im Vakuum konzentriert, mit Diäthyläther versetzt und vom Niederschlag abgetrennt. Das Filtrat wird in Aceton aufgenommen und mit Wasser versetzt. Der farblose Niederschlag wird abfiltriert und getrocknet. Man erhält die Verbindung der Formel

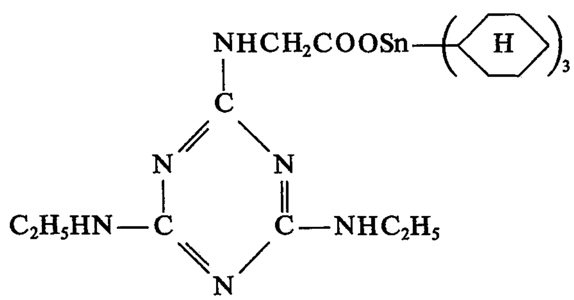

(Verbindung E)

mit einem Schmelzpunkt von 95–98°C.

Auf analoge Weise, aber mit dem unter b) hergestellten Zwischenprodukt, erhält man auch die Verbindung der Formel

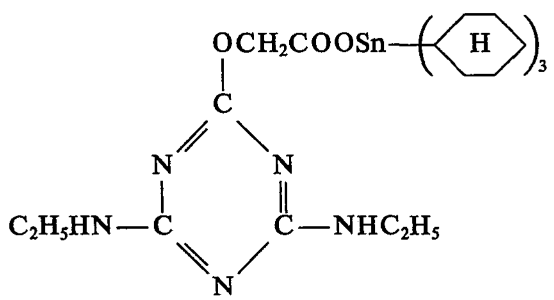

(Verbindung F)

mit einem Schmelzpunkt von 72—75° C.

Beispiel 3

Wirkung gegen pflanzenschädigende Akariden:
Tetranychus urticae (OP-sensibel) und Tetranychus cinnabarinus (OP-resistent)

Die Primärblätter von Phaseolus-vulgaris-Pflanzen wurden 16 Stunden vor Versuchsbeginn mit infestierten Blattstücken aus Massenzuchten von Tetranychus urtica (OP-sensibel) und Tetranychus cinnabarinus (OP-resistent) belegt. Dabei wurde für jede Spezies (und Konzentration) eine Pflanze eingesetzt. Nach Entfernung der Blattstücke befanden sich auf den Pflanzen sowohl Eier wie Larven und Adulte der Testmilben. Die Behandlung mit dem Wirkstoff erfolgte durch Direktspray bis zur Tropfnässe in einer Verdünnungsreihe, die aus einem 25%igen Emulsionskonzentrat hergestellt wurde und Konzentrationen von 50, 100, 200 und 400 ppm Wirkstoff aufwies. Die so behandelten Pflanzen wurden während der ganzen Versuchsdauer in Gewächshauskabinen bei 25°C gehalten. Die Auswertung wurde 2 und 7 Tage nach erfolgter Wirkstoffapplikation vorgenommen, wobei die Mortalität von Eiern (7 Tage), Larven und Adulten (2 und 7 Tage) festgestellt und in Prozenten angegeben wird.

| Verbindung | Stadium | Tetranychus urticae | | | | Tetranychus cinnabarinus | | | |
|---|---|---|---|---|---|---|---|---|---|
| | | 2 d | | 7 d | | 2 d | | 7 d | |
| | | W | K | W | K | W | K | W | K |
| A | Adulte | 100 | 100 | 100 | 50 | 100 | 100 | 100 | 100 |
| | Larven | 100 | 100 | 100 | 50 | 100 | 100 | 100 | 100 |
| | Eier | | | 80—99 | 400 | | | 80—99 | 400 |
| B | Adulte | 100 | 100 | 100 | 100 | 100 | 100 | 100 | 100 |
| | Larven | 100 | 100 | 100 | 100 | 100 | 100 | 100 | 100 |
| | Eier | | | 80—99 | 200 | | | 60—79 | 400 |

W = Wirkung in %.
K = Konzentration in ppm.

**Patentansprüche**

1. Eine Triorganozinn-Triazinverbindung der Formel

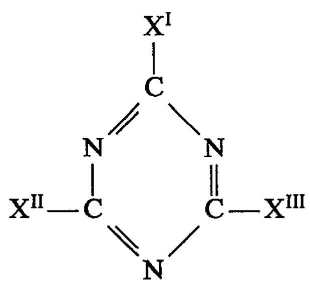

(I)

worin

$X^I$ $-S-Y-COOSn(R')_3$, $-O-Y-COOSn(R')_3$ oder $-NH-Y-COOSn(R')_3$,
$X^{II}$ und $X^{III}$ je $X^I$, Halogen, $(C_1-C_6\text{-Alkyl})_2N-$, $(C_1-C_6\text{-Alkyl})-NH-$, $(C_1-C_6\text{-Alkyl})-O-$ der $(C_1-C_6\text{-Alkyl})-S-$,
Y $C_1-C_6$-Alkylen und die Reste

R' gleich oder verschieden sind und $C_1-C_{12}$-Alkyl oder gegebenenfalls substituiertes Benzyl, Phenyl oder Cyclohexyl bedeuten.

2. Eine Verbindung gemäß Anspruch 1, worin

$X^I$ $-S-Y-COOSn(R')_3$,
$X^{II}$ und $X^{III}$ je ($C_1-C_6$-Alkyl)NH—,
Y Methylen und
R' n-Butyl, Phenyl oder Cyclohexyl bedeuten.

3. Eine Verbindung gemäß Anspruch 1, worin

$X^I$ $-S-Y-COOSn(R')_3$,
$X^{II}$ und $X^{III}$ je ($C_1-C_6$-Alkyl)NH—,
Y Methylen und
R' Cyclohexyl bedeuten.

4. Die Verbindung gemäß Anspruch 3 der Formel

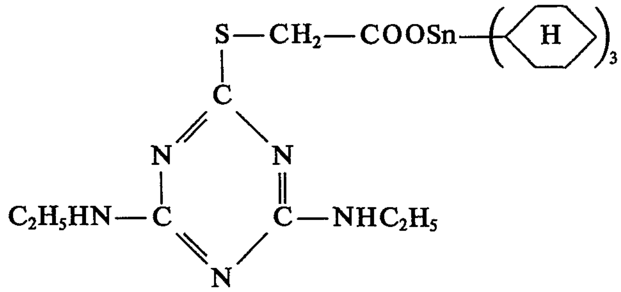

5. Die Verbindung gemäß Anspruch 3 der Formel

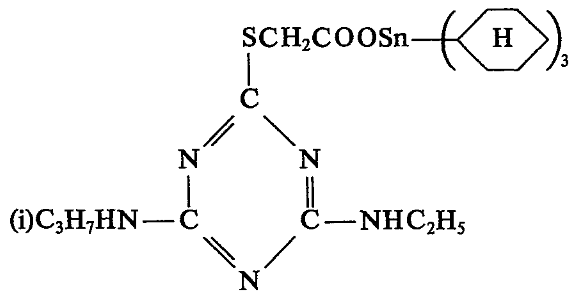

6. Die Verbindung gemäß Anspruch 2 der Formel

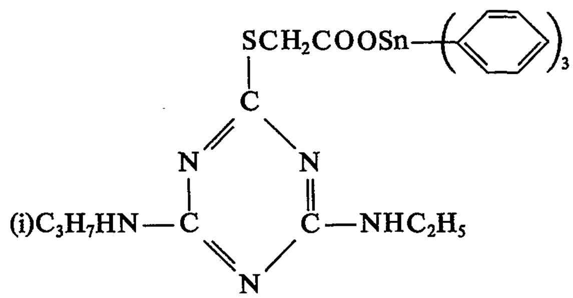

7. Die Verbindung gemäß Anspruch 2 der Formel

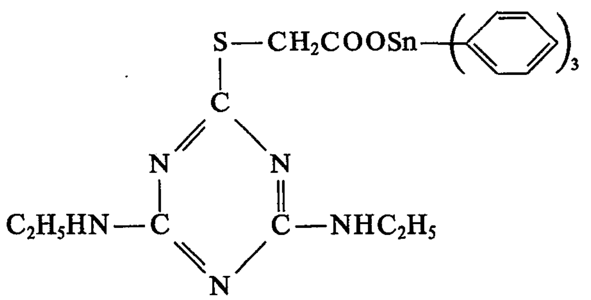

8. Verfahren zur Herstellung von Verbindungen gemäß Anspruch 1, dadurch gekennzeichnet, daß man a) eine Verbindung der Formel

$X^{II}$ oder 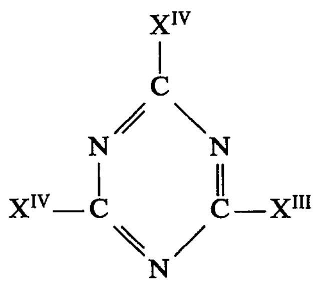 oder $X^{IV}$

mit einer Verbindung der Formel $XSn(R')_3$ umsetzt, worin $X^{II}$, $X^{III}$ und R′ die im Anspruch 1 angegebene Bedeutung haben, $X^{IV}$ für $-S-Y-COOH$, $-O-Y-COOH$ oder $-NH-Y-COOH$, worin Y $C_1-C_6$-Alkylen bedeutet, und X für eine $(R')_3SnO$- oder HO-Gruppe oder ein Halogenatom steht.

9. Ein Schädlingsbekämpfungsmittel, welches als aktive Komponente eine Verbindung gemäß Anspruch 1 enthält.

10. Verwendung einer Verbindung gemäß Anspruch 1 zur Bekämpfung von Schädlingen an Tieren und Pflanzen.

11. Verwendung einer Verbindung gemäß Anspruch 1 zur Bekämpfung von Vertretern der Ordnung Akarina.

12. Eine Verbindung der Formel

$X^{II}$ oder 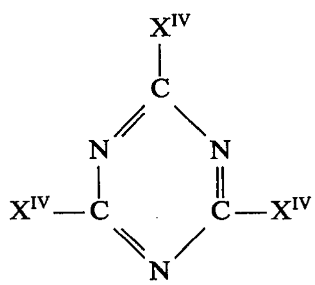 oder $X^{III}$ (II)

worin $X^{II}$ und $X^{III}$ Halogen, die Reste $-S-Y-COOSn(R')_3$, $-O-Y-COOSn(R')_3$, $-NH-Y-COOSn(R')_3$, $(C_{1-6}\text{-Alkyl})_2N-$, $C_{1-6}$-Alkyl$-NH-$, $C_{1-6}$-Alkyl$-O-$ oder $C_{1-6}$-Alkyl$-S-$ bedeuten, $X^{IV}$ für $-S-Y-COOH$, $-O-Y-COOH$ oder $-NH-Y-COOH$ und Y für $C_{1-6}$-Alkylen stehen.

13. Eine Verbindung gemäß Anspruch 12, welche die Reste $X^{II}$, $X^{III}$ und $X^{IV}$ enthält.

**Claims**

1. A triorgano-tin triazine compound of the formula

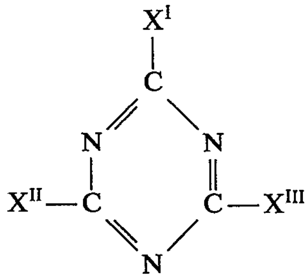

(I)

wherein $X^{I}$ is $-S-Y-COOSn(R')_3$, $-O-Y-COOSn(R')_3$ or $-NH-Y-COOSn(R')_3$, each of $X^{II}$ and $X^{III}$ has the same meaning as $X^{I}$ or is halogen, $(C_1-C_6alkyl)_2N-$, $(C_1-C_6alkyl)-NH-$, $(C_1-C_6alkyl)-O-$ or $(C_1-C_6alkyl)-S-$, Y is $C_1-C_6$alkylene, and the radicals R′ can be the same or different and are $C_1-C_{12}$alkyl or unsubstituted or substituted benzyl, phenyl or cyclohexyl.

2. A compound according to claim 1, wherein $X^{I}$ is $-S-Y-COOSn(R')_3$, each of $X^{II}$ and $X^{III}$ is $(C_1-C_6alkyl)-NH-$, Y is methylene and R′ is n-butyl, phenyl or cyclohexyl.

3. A compound according to claim 1, wherein $X^{I}$ is $-S-Y-COOSn(R')_3$, each of $X^{II}$ and $X^{III}$ is $(C_1-C_6alkyl)-NH-$, Y is methylene and R′ is cyclohexyl.

4. The compound according to claim 3 of the formula

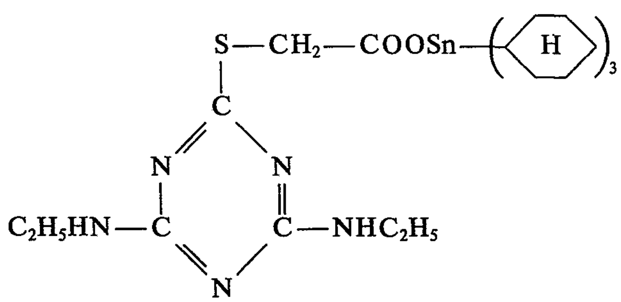

5. The compound according to claim 3 of the formula

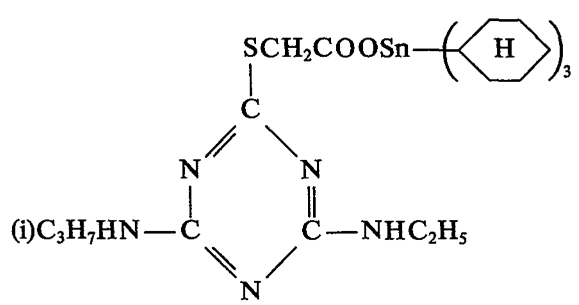

6. The compound according to claim 2 of the formula

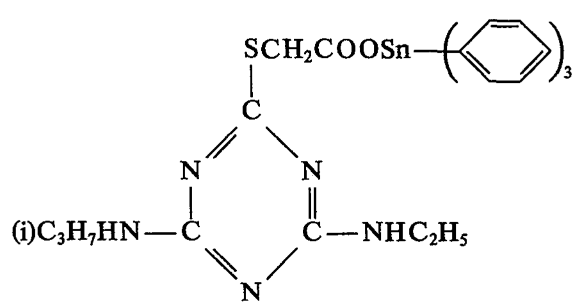

7. The compound according to claim 2 of the formula

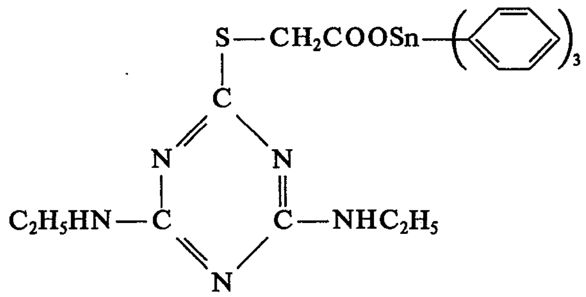

8. A process for the production of a compound according to claim 1, which process comprises reacting a compound of the formula

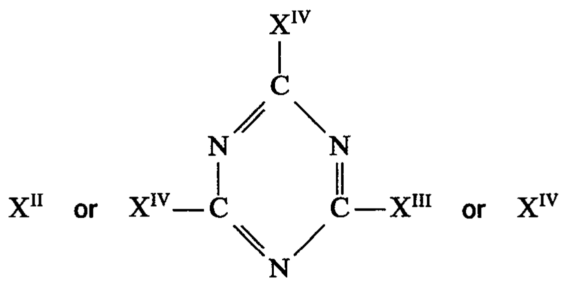

with a compound of the formula $XSn(R')_3$, in which formulae $X^{II}$, $X^{III}$ and R′ are as defined in claim 1, $X^{IV}$ is —S—Y—COOH, —O—Y—COOH or —NH—Y—COOH, wherein Y is $C_1$—$C_6$alkylene and X is a $(R')_3SnO$ or HO group or is a halogen atom.

9. A pesticidal composition which contains, as active component, a compound according to claim 1.

10. A method of controlling pests of animals and plants at a locus, which comprises applying to said locus a pesticidally effective amount of a compound according to claim 1.

11. A method according to claim 1, wherein the pests to be controlled are representatives of the order Acarina.

12. A compound of the formula

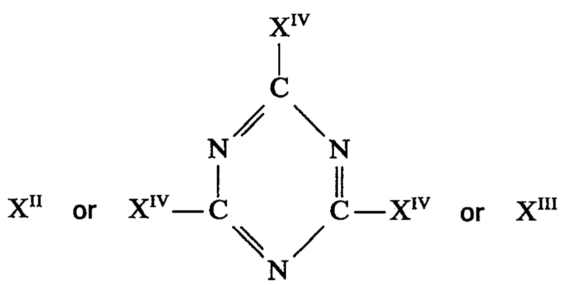

wherein $X^{II}$ and $X^{III}$ are halogen, the radicals —S—Y—$COOSn(R')_3$, —O—Y—$COOSn(R')_3$, —NH—Y—$COOSn(R')_3$, $(C_1$—$C_6alkyl)_2N$—, $(C_1$—$C_6alkyl)$—NH, $(C_1$—$C_6alkyl)$—O— or $(C_1$—$C_6alkyl)$—S—, $X^{IV}$ is —S—Y—COOH, O—Y—COOH or —NH—Y—COOH, and Y is $C_1$—$C_6$alkylene.

13. A compound according to claim 12, which contains the radicals $X^{II}$, $X^{III}$ and $X^{IV}$.

**Revendications**

1. Composé de triazine contenant des groupements de triorgano-etain de formule

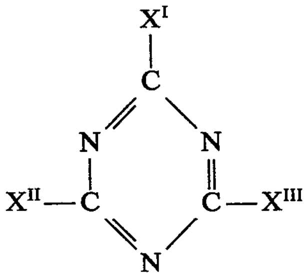

(I)

où

$X^I$ représente $-S-Y-COOSn(R')_3$, $-O-Y-COOSn(R')_3$ ou $-NH-Y-COOSn(R')_3$,
$X^{II}$ et $X^{III}$ représentent chacun $X^I$, un halogène, un (alcoyle en $C_1$ à $C_6)_2N-$, un (alcoyle en $C_1$ à $C_6)-NH-$, un (alcoyle en $C_1$ à $C_6)-O-$ ou un (alcoyle en $C_1$ à $C_6)-S-$,
Y représente un alcoylène en $C_1$ à $C_6$ et les radicaux
R' sont semblables ou différents et représentent un alcoyle en $C_1$ à $C_{12}$ ou un benzyle, un phényle ou un cyclohexyle éventuellement substitué.

2. Composé selon la revendication 1, où

$X^I$ représente $-S-Y-COOSn(R')_3$,
$X^{II}$ et $X^{III}$ représentent chacun un (alcoyle en $C_1$ à $C_6)-NH-$,
Y représente un méthylène et
R' représente un n-butyle, un phényle ou un cyclohexyle.

3. Composé selon la revendication 1, où

$X^I$ représente $-S-Y-COOSn(R')_3$,
$X^{II}$ et $X^{III}$ représentant chacun un (alcoyle en $C_1$ à $C_6)-NH-$,
Y représente un méthylène et
R' représente un cyclohexyle.

4. Composé selon la revendication 3 de formule

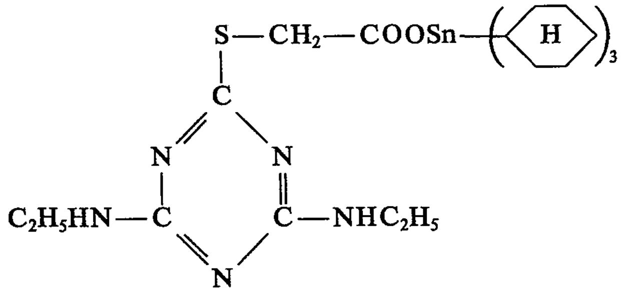

5. Composé selon la revendication 3 de formule

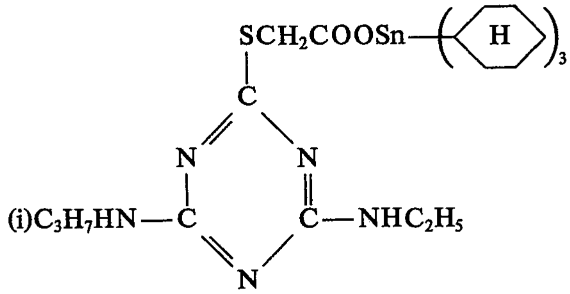

6. Composé selon la revendication 2 de formule

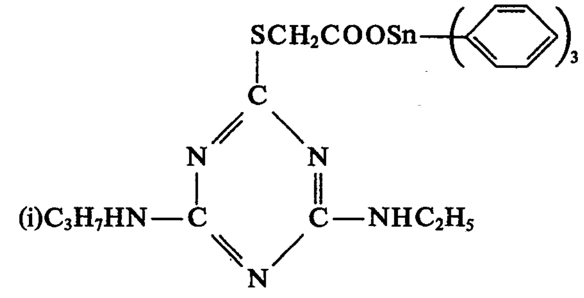

7. Composé selon la revendication 2 de formule

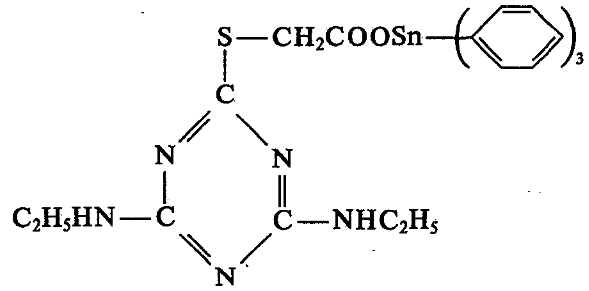

8. Procédé de préparation de composés selon la revendication 1, caractérisé en ce que a) on fait réagir un composé de formule

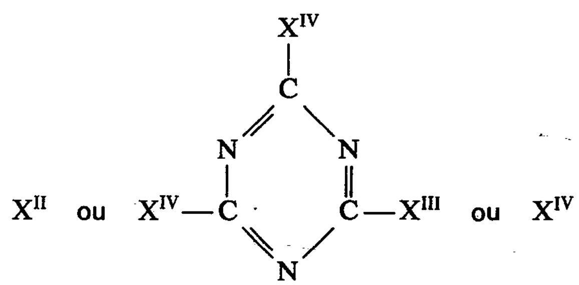

avec un composé de formule $XSn(R')_3$ où $X^{II}$, $X^{III}$ et R′ ont la signification donnée dans la revendication 1, $X^{IV}$ représente —S—Y—COOH, —O—Y—COOH ou —NH—Y—COOH, où Y représente un alcoylène en $C_1$ à $C_6$, et X représente un groupe $(R')_3SnO$ ou HO ou un atome d'halogène.

9. Agent de lutte antiparasitaire contenant comme composant actif un composé actif un composé selon la revendication 1.

10. Application d'un composé selon la revendication 1 à la lutte contre les parasites chez les animaux et les plantes.

11. Application d'un composé selon la revendication 1 à la lutte contre les représentants de l'ordre des acariens.

12. Composé de formule

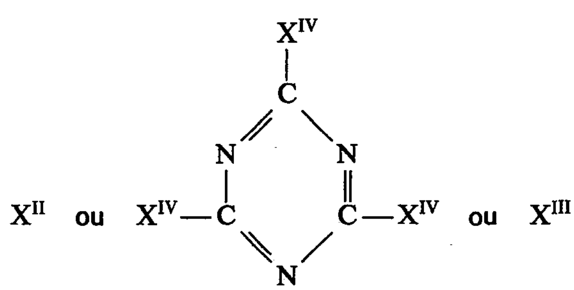

(II)

où $X^{II}$ et $X^{III}$ représentent un halogène, les radicaux $-S-Y-COOSn(R')_3$, $-O-Y-COOSn(R')_3$, $-NH-Y-COOSn(R')_3$, (alcoyle en $C_1$ à $C_6$)$_2$N—, alcoyle en $C_1$ à $C_6$—NH—, alcoyle en $C_1$ à $C_6$—O— ou alcoyle en $C_1$ à $C_6$—S—, $X^{IV}$ représente $-S-Y-COOH$, $-O-Y-COOH$ ou $-NH-Y-COOH$ et Y représente un alcoylène en $C_1$ à $C_6$.

13. Composé selon la revendication 12, qui contient les radicaux $X^{II}$, $X^{III}$ et $X^{IV}$.